# EUROPEAN PATENT APPLICATION

(11) **EP 2 992 809 A1**
(43) Date of publication of application: **09.03.2016**
(21) Application number: 14823647.4
(22) Date of filing: 31.03.2014
(51) Int. Cl.: A61B 1/04, G02B 23/24

(54) **ENDOSCOPE SYSTEM**

(30) Priority: 12.07.2013 JP 2013146852
(71) Applicant: OLYMPUS CORPORATION, Shibuya-ku Tokyo 151-0072 (JP)
(72) Inventor: IMAIZUMI Katsuichi, Tokyo 151-0072 (JP); TAKEYAMA Tetsuhide, Tokyo 151-0072 (JP); SAKAI Yuji, Tokyo 151-0072 (JP); OKITA Yoshinari, Tokyo 151-0072 (JP)
(74) Representative: Gunzelmann, Rainer
(86) International application number: PCT/JP2014/059452
(87) International publication number: WO 2015/004960

(57) **Abstract**

An endoscope system includes: an endoscope configured to have a field of view in front of a distal end portion of an insertion portion having flexibility and an elongated shape; an insertion assisting instrument including a conduit having an inner portion through which the insertion portion can be inserted, and the insertion assisting instrument being configured to enable an angle of a distal end portion of the conduit to be changed; an image generation section that generates an image corresponding to the field of view of the endoscope and outputs the generated image; a rotation angle calculation section that calculates a rotation angle indicating to what extent an orientation of the image is rotated with respect to a reference direction, the image being outputted from the image generation section when at least a part of the insertion portion is inserted through the conduit; and an image rotation section that performs image rotation processing on the image outputted from the image generation section, for displaying, on a display screen of a display section, the image with the rotation angle being offset.

## Description

### Technical Field

The present invention relates to an endoscope system and more particularly to an endoscope system including an insertion assisting instrument.

### Background Art

In medical fields, an insertion assisting instrument for assisting insertion of an endoscope into a deep part of a body cavity has been conventionally known, and such an insertion assisting instrument is used with the endoscope being inserted in a predetermined conduit of the insertion assisting instrument.

Specifically, for example, U.S. Patent Application Publication No. 2011/0213300 discloses, as an instrument similar to the above-described insertion assisting instrument, a movable catheter assembly including (an imaging device port and) a working channel through which an endoscope, etc. is inserted, and configured to enable an angle of a catheter distal end portion to be changed in up and down directions and right and left directions in response to an operation of a knob.

However, the configuration disclosed in the U.S. Patent Application Publication No. 2011/0213300 has such a problem as a frequent occurrence of a situation where a direction in which the angle of the catheter distal end portion is changed and a direction in which the field of view of the endoscope moves in accordance with the change of the angle of the catheter distal end portion are different from each other, when the knob is operated with the endoscope being inserted through the working channel.

Therefore, the configuration disclosed in the U.S. Patent Application Publication No. 2011/0213300 has such a problem to be solved, which arises from the above-described problem, as degradation of operability when the movable catheter assembly is used with the endoscope being inserted in the working channel.

The present invention has been achieved in view of the above-described circumstances, and an object of the present invention is to provide an endoscope system capable of improving operability at the time of changing an angle of an insertion assisting instrument used with the endoscope being inserted therethrough.

### Disclosure of Invention

### Means for Solving the Problem

An endoscope system according to one aspect of the present invention includes: an endoscope including an insertion portion formed so as to have flexibility and an elongated shape, and configured to have a field of view in front of a distal end portion of the insertion portion; an insertion assisting instrument including a conduit having an inner portion through which the insertion portion can be inserted, and the insertion assisting instrument being configured to enable an angle of a distal end portion of the conduit to be changed in response to an operation of an angle operation portion; an image generation section configured to generate an image corresponding to the field of view of the endoscope and output the generated image; a rotation angle calculation section configured to calculate a rotation angle indicating to what extent an orientation of the image is rotated with respect to a reference direction, the image being outputted from the image generation section when at least a part of the insertion portion is inserted through the conduit; and an image rotation section configured to perform image rotation processing on the image outputted from the image generation section, for displaying, on a display screen of a display section, the image with the rotation angle being offset.

### Brief Description of the Drawings

FIG 1 illustrates a main part of an endoscope system according to a first embodiment.
FIG. 2 is a block diagram for illustrating one example of a main body apparatus according to the first embodiment.
FIG. 3 illustrates one example of an image before image rotation processing according to the first embodiment is performed.
FIG. 4 illustrates one example of an image displayed after the image rotation processing according to the first embodiment has been performed.
FIG. 5 illustrates a configuration of a main part of an endoscope system according to a second embodiment.
FIG. 6 is a block diagram for illustrating one example of a configuration of a main body apparatus according to the second embodiment.
FIG. 7 illustrates one example of an image and a character string displayed when the endoscope system according to the second embodiment is used.
FIG. 8 illustrates one example of an image before an image rotation processing according to the second embodiment is performed.
FIG. 9 illustrates one example of an image and a character string displayed after the image rotation processing according to the second embodiment has been performed.
FIG. 10 illustrates a configuration of a main part of an endoscope system according to a third embodiment.
FIG. 11 is a block diagram for illustrating one example of a configuration of a main body apparatus according to the third embodiment.
FIG. 12 illustrates a configuration of a main part of an endoscope system according to a fourth embodiment.
FIG. 13 is a block diagram for illustrating one example of a configuration of a main body apparatus according to the fourth embodiment.

### Best Mode for Carrying Out the Invention

Hereinafter, description will be made on embodiments of the present invention with reference to drawings.

### (First Embodiment)

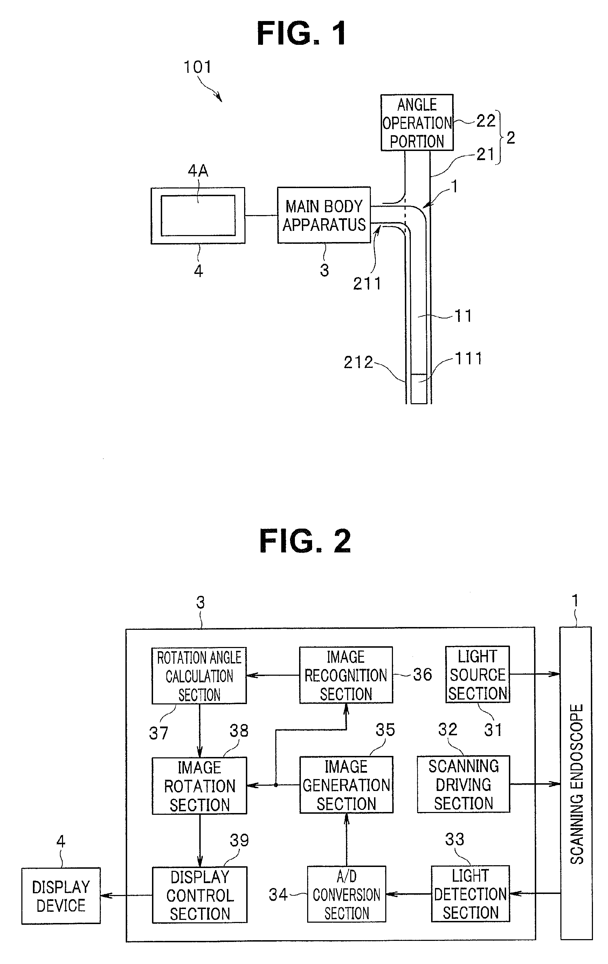

FIGS. 1 to 4 relate to the first embodiment of the present invention. FIG 1 illustrates a configuration of a main part of an endoscope system according to the first embodiment.

As shown in FIG. 1, an endoscope system 101 includes a scanning endoscope 1, an insertion assisting instrument 2, a main body apparatus 3, and a display device 4.

The scanning endoscope 1 is configured by a member made of resin having flexibility, or the like, and includes an insertion portion 11 formed so as to have an elongated shape insertable into a body cavity of a subject to be examined.

The insertion portion 11 includes, at the proximal end portion thereof, a connector (not shown) for detachably connecting the scanning endoscope 1 to the main body apparatus 3. In addition, the insertion portion 11 includes: a light-guiding portion (not shown) including an optical fiber for guiding illumination light supplied from the main body apparatus 3 to a distal end portion 111; a light condensing optical system (not shown) configured to condense the illumination light guided by the light-guiding portion to emit the condensed illumination light toward an object in front of the distal end portion 111; and a light-receiving portion (not shown) including a fiber bundle for receiving the return light from the object at the distal end portion 111 to guide the received return light to the main body apparatus 3. In addition, the insertion portion 11 includes, at the distal end portion 111, an actuator (not shown) which includes a plurality of piezoelectric elements that vibrate in response to the driving signal supplied from the main body apparatus 3 and which is configured to allow a light-emission-side end portion of the light-guiding portion to oscillate with the vibration of the plurality of piezoelectric elements.

That is, the scanning endoscope 1 is configured to have a field of view in front of the distal end portion 111 of the insertion portion 11 (to be able to obtain an optical image by scanning an object which is present in front of the distal end portion 111 of the insertion portion 11).

As shown in FIG. 1, the insertion assisting instrument 2 includes a flexible tube portion 21 and an angle operation portion 22.

The flexible tube portion 21 is made of resin having flexibility, or the like, and includes an insertion port 211 from which the insertion portion 11 can be inserted. In addition, the flexible tube portion 21 is formed as a conduit having an inner portion through which the insertion portion 11 can be inserted and allowing the distal end portion 111 of the insertion portion 11 to be protruded from the distal end portion 212. Furthermore, the flexible tube portion 21 is provided with bending pieces, wires, etc., for causing a bending portion (not shown) adjacent to the distal end portion 212 to bend.

The angle operation portion 22 includes, for example, an operation device such as knob, lever, or the like, and is configured to be able to change the angle of the distal end portion 212 in up and down directions and right and left directions by causing the bending portion of the flexible tube portion 21 to bend in accordance with the operation by the user.

FIG. 2 is a block diagram for illustrating one example of a main body apparatus according to the first embodiment.

As shown in FIG. 2, the main body apparatus 3 includes a light source section 31, a scanning driving section 32, a light detection section 33, an A/D conversion section 34, an image generation section 35, an image recognition section 36, a rotation angle calculation section 37, an image rotation section 38, and a display control section 39.

The light source section 31 is provided with a laser light source and the like, for example, and supplies illumination light for illuminating an object to the light-guiding portion of the scanning endoscope 1.

The scanning driving section 32 generates a driving signal for oscillating the light-emission-side end portion of the light-guiding portion of the scanning endoscope 1 in a predetermined scanning pattern (in a spiral shape, etc., for example) and supplies the generated driving signal to the actuator of the scanning endoscope 1.

The light detection section 33 generates an electric signal corresponding to the return light received by the light-receiving portion of the scanning endoscope 1, and outputs the generated electric signal to the A/D conversion section 34.

The A/D conversion section 34 converts the electric signal outputted from the light detection section 33 into a digital signal, and outputs the digital signal to the image generation section 35.

The image generation section 35 performs processing such as two-dimensional mapping on the digital signal outputted in a time-series manner from the A/D conversion section 34, to generate an image corresponding to the field of view of the scanning endoscope 1 and output the generated image to the image recognition section 36 and image rotation section 38.

The image recognition section 36 performs image recognition processing on the image outputted from the image generation section 35, to thereby be capable of determining whether or not a predetermined mark is included in the image. When obtaining the determination result that the predetermined mark is included in the image outputted from the image generation section 35, the image recognition section 36 outputs the image to the rotation angle calculation section 37.

The rotation angle calculation section 37 calculates a rotation angle which indicates to what extent the orientation of the image which is generated by the image generation section 35 when at least a part of the insertion portion 11 is inserted in the flexible tube portion 21 is rotated with respect to the reference direction. Specifically, the rotation angle calculation section 37 performs, for example, processing for calculating an angle θ1 indicating to what extent the position of the predetermined mark which is included in the image outputted from the image generation section 35 through the image recognition section 36 is rotated with respect to the reference direction to be described later. In addition, the rotation angle calculation section 37 outputs the calculation result of the angle θ1 obtained by the above-described processing to the image rotation section 38.

When the angle θ1 is outputted from the rotation angle calculation section 37, the image rotation section 38 performs image rotation processing on the image outputted from the image generation section 35 for displaying, on a display screen 4A of the display device 4, the image with the rotation angle θ1 being offset. In other words, the image rotation section 38 performs, on the basis of the angle θ1 outputted from the rotation angle calculation section 37, image rotation processing for rotating the image outputted from the image generation section 35 by negative θ1. Then, the image rotation section 38 outputs the image subjected to the above-described image rotation processing to the display control section 39.

The display control section 39 performs processing on the image outputted from the image rotation section 38 for adapting the display format of the image to a predetermined display format, and outputs the image subjected to the processing to the display device 4.

The display device 4 is provided with a monitor and the like, for example, and configured to be able to display the image outputted from the main body apparatus 3, and the like, on the display screen 4A.

Next, description will be made on the working of the endoscope system 101 according to the present embodiment.

The user inserts the insertion portion 11 into the inner portion of the flexible tube portion 21 from the insertion port 211 of the insertion assisting instrument 2 in the state where the scanning of the object by the scanning endoscope 1 and image generation by the main body apparatus 3 are started.

In the present embodiment, on the inner wall of the flexible tube portion 21, the predetermined mark is drawn for enabling the recognition that the angle of the distal end portion 212 is changed in a predetermined direction when the bending portion of the flexible tube portion 21 is bent in response to the operation of the angle operation portion 22. Specifically, on the inner wall of the flexible tube portion 21 of the present embodiment, a green identifying line is drawn for enabling the recognition that the angle of the distal end portion 212 is changed in the up direction when the bending portion of the flexible tube portion 21 is bent in response to the operation of the angle operation portion 22, for example.

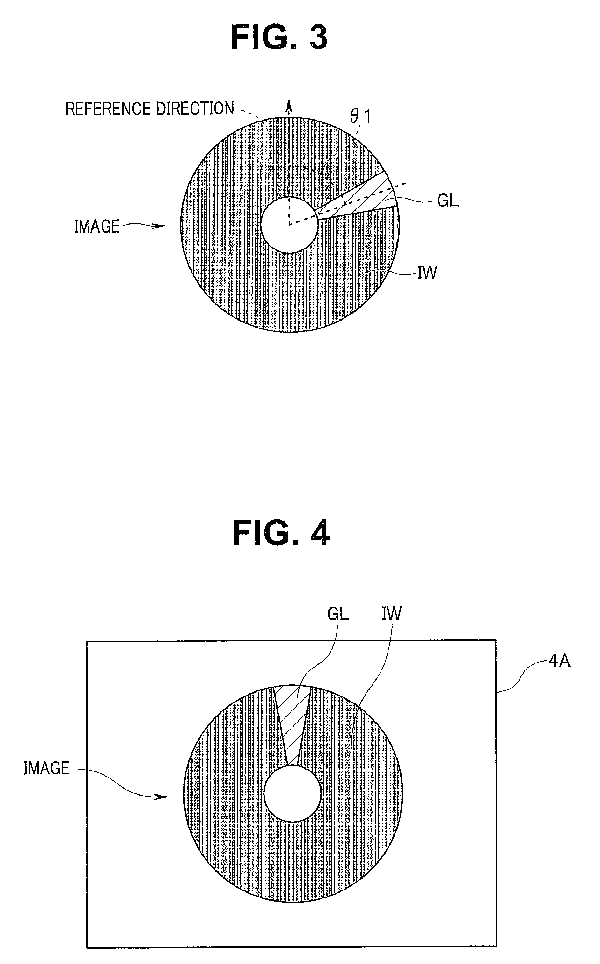

Therefore, when the distal end portion 111 of the insertion portion 11 is located at the inner portion of the flexible tube portion 21 (while the insertion portion 11 is inserted in the flexible tube portion 21), the image including the inner wall IW of the flexible tube portion 21 and the green identifying line GL drawn on the inner wall IW, as exemplified in FIG 3, is outputted from the image generation section 35. FIG. 3 illustrates one example of the image before image rotation processing according to the first embodiment is performed.

The image recognition section 36 performs image recognition processing on the image outputted from the image generation section 35, to thereby determine whether or not the green identifying line GL is included in the image. When obtaining the determination result that the green identifying line GL is included in the image outputted from the image generation section 35, the image recognition section 36 outputs the image to the rotation angle calculation section 37.

The rotation angle calculation section 37 performs processing for calculating the angle θ1 indicating to what extent the position of the green identifying line GL included in the image outputted from the image recognition section 36 is rotated with respect to the up direction (reference direction) of the display screen 4A (see FIG 3). In addition, the rotation angle calculation section 37 outputs the calculation result of the angle θ1 obtained by the above-described processing to the image rotation section 38.

The image rotation section 38 performs, on the basis of the angle θ1 outputted from the rotation angle calculation section 37, image rotation processing for rotating the image outputted from the image generation section 35 by negative θ1. Such image rotation processing is performed on the image as shown in FIG. 3, and as a result, the image in which the green identifying line GL matches with the up direction (reference direction) on the display screen 4A, as shown in FIG. 4, is displayed (on the display screen 4A). FIG 4 illustrates one example of an image displayed after the image rotation processing according to the first embodiment has been performed.

As described above, according to the endoscope system 101 of the present embodiment, when the insertion assisting instrument is used with the insertion portion 11 being inserted through the inner portion of the flexible tube portion 21, it is possible to cause the direction in which the angle of the distal end portion 212 is changed in response to the operation of the angle operation portion 22 to match with the direction in which the field of view of the scanning endoscope 1 moves as the angle of the distal end portion 212 is changed. That is, according to the present embodiment, it is possible to improve the operability at the time of changing the angle of the insertion assisting instrument used with the endoscope being inserted therethrough.

### (Second Embodiment)

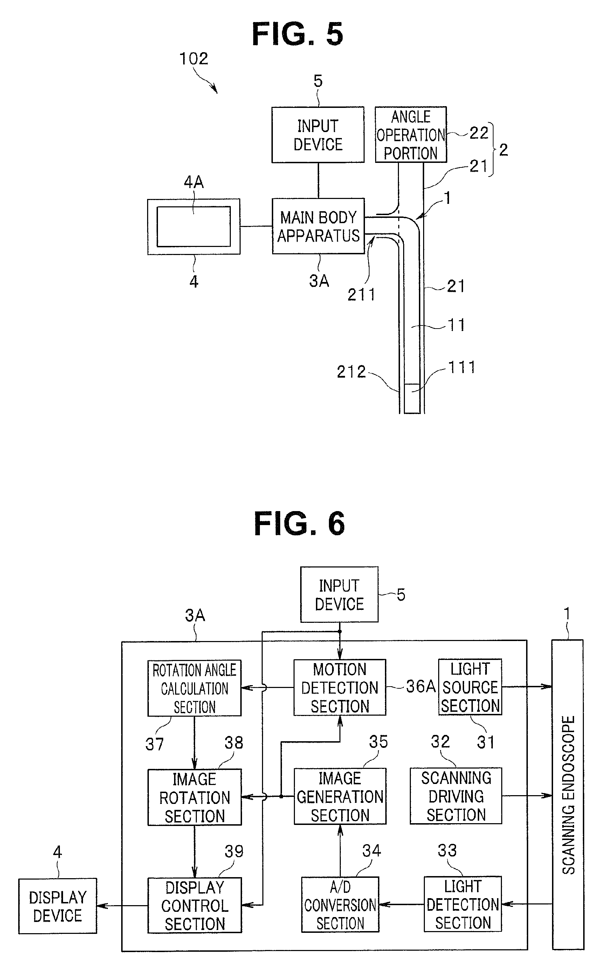

FIGS. 5 to 9 relate to the second embodiment of the present invention. FIG. 5 illustrates a configuration of a main part of an endoscope system according to the second embodiment.

Note that, in the present embodiment, detailed description on the parts having configurations same as those in the first embodiment will be appropriately omitted and description will be mainly made on the parts having configurations different from those in the first embodiment.

The endoscope system 102 includes a scanning endoscope 1, an insertion assisting instrument 2, a main body apparatus 3A, a display device 4, and an input device 5, as shown in FIG. 5.

The input device 5 includes a user interface such as buttons and/or switches, and is configured to be able to give various instructions to the main body apparatus 3A in response to the operation by the user.

FIG. 6 is a block diagram for illustrating one example of a configuration of a main body apparatus according to the second embodiment.

The main body apparatus 3A includes a light source section 31, a scanning driving section 32, a light detection section 33, an A/D conversion section 34, an image generation section 35, a motion detection section 36A, a rotation angle calculation section 37, an image rotation section 38, and a display control section 39, as shown in FIG. 6.

The image generation section 35 performs processing such as two-dimensional mapping on the digital signal outputted from the A/D conversion section 34 in a time-series manner, to generate an image corresponding to the field of view of the scanning endoscope 1, and outputs the generated image to the motion detection section 36A and the image rotation section 38.

The motion detection section 36A performs processing such as pattern recognition or template matching by using the images sequentially outputted from the image generation section 35 during the period after the detection of the depression of a calibration switch (not shown) on the input device 5 until a predetermined time period has elapsed, to thereby obtain the motion vector of the object included in the images. In addition, the motion detection section 36A outputs the motion vector obtained by the above-described processing to the rotation angle calculation section 37.

The rotation angle calculation section 37 performs processing for calculating an angle θ2 indicating to what extent the motion vector outputted from the motion detection section 36A is rotated with respect to the reference direction to be described later. In addition, the rotation angle calculation section 37 outputs the calculation result of the angle θ2 obtained by the above-described processing to the image rotation section 38.

When the angle θ2 is outputted from the rotation angle calculation section 37, the image rotation section 38 performs image rotation processing on the image outputted from the image generation section 35, for displaying, on the display screen 4A of the display device 4, the image with the angle θ2 being offset. In other words, the image rotation section 38 performs, on the basis of the angle θ2 outputted from the rotation angle calculation section 37, image rotation processing for rotating the image outputted from the image generation section 35 by negative θ2.

When detecting that the calibration switch on the input device 5 has been depressed, the display control section 39 generates a character string to urge the user to perform the operation for changing the angle of the distal end portion 212 in a predetermined direction, and outputs the generated character string to the display device 4. In addition, the display control section 39, when detecting that the image subjected to the above-described image rotation processing has been outputted from the image rotation section 38 within a predetermined period after the detection of the depression of the calibration switch on the input device 5, generates a character string indicating the completion of the calibration operation started by the depression of the calibration switch, to output the generated character string to the display device 4.

Next, description will be made on the working of the endoscope system 102 according to the present embodiment.

The user inserts the insertion portion 11 into the inner portion of the flexible tube portion 21 from the insertion port 211 of the insertion assisting instrument 2 in the state where the scanning of the object by the scanning endoscope 1 and the image generation by the main body apparatus 3 are started. Then, the user causes the distal end portion 111 to protrude from the distal end portion 212, to thereby confirm that the image obtained by scanning an arbitrary object is displayed on the display device 4, and thereafter depresses the calibration switch on the input device 5.

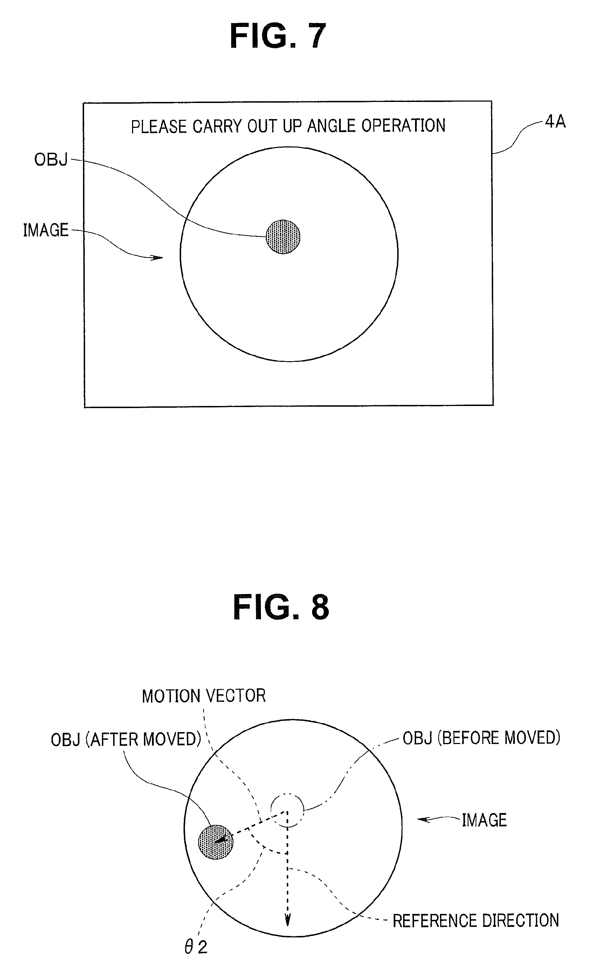

When detecting that the calibration switch on the input device 5 has been depressed, the display control section 39 generates a character string for urging the user to perform the operation for changing the angle of the distal end portion 212 in the predetermined direction, to output the generated character string to the display device 4. Then, in accordance with such operation of the display control section 39, the image including the object OBJ and the character string ("Please carry out an UP angle operation") for urging the user to perform the operation for changing the angle of the distal end portion 212 in the predetermined direction are displayed together on the display screen 4A, as shown in FIG. 7, for example. FIG. 7 illustrates one example of the image and the character string displayed when the endoscope system according to the second embodiment is used.

After that, the user operates the angle operation portion 22 on the basis of the character string displayed on the display screen 4A, to change the angle of the distal end portion 212 in the predetermined direction. Note that, hereinafter, for simplification, description will be made by taking the case where the operation for changing the angle of the distal end portion 212 in the up direction has been performed, as an example.

The motion detection section 36A performs processing such as pattern recognition or template matching on the basis of the temporal change of the position of the object OBJ included in the images sequentially outputted from the image generation section 35 during the period after the detection of the depression of the calibration switch on the input device 5 until a predetermined period has elapsed, to thereby obtain the motion vector of the object OBJ and output the obtained motion vector to the rotation angle calculation section 37.

The motion vector (the moving direction of the object OBJ in accordance with the change of the angle of the distal end portion 212) obtained by the motion detection section 36A is supposed to be a direction opposite to the moving direction of the field of view of the scanning endoscope 1 (see FIG 8). Therefore, the rotation angle calculation section 37 performs processing for calculating the angle θ2 indicating to what extent the motion vector outputted from the motion detection section 36A is rotated with respect to the down direction (reference direction) on the display screen 4A (see FIG. 8). FIG. 8 illustrates one example of the image before the image rotation processing according to the second embodiment is performed.

Note that, according to the present embodiment, the reference direction used for calculating the angle θ2 may be another direction other than the down direction on the display screen 4A, as long as the reference direction is a direction opposite to the motion vector obtained by the motion detection section 36A.

The image rotation section 38 performs, on the basis of the angle θ2 outputted from the rotation angle calculation section 37, image rotation processing for rotating the image outputted from the image generation section 35 by negative θ2.

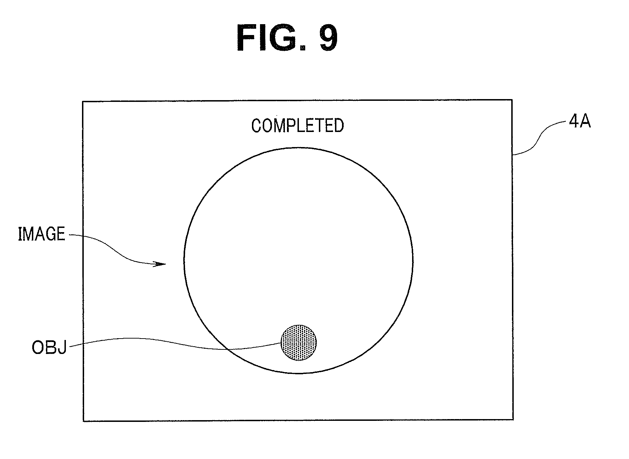

The display control section 39, when detecting that the image subjected to the above-described image rotation processing has been outputted from the image rotation section 38 within a predetermined period after the detection of the depression of the calibration switch on the input device 5, generates a character string indicating the completion of the calibration operation started by the depression of the calibration switch, to output the generated character string to the display device 4. In response to the operation of the display control section 39, the image subjected to the image rotation processing by the image rotation section 38 and the character string ("Completed") indicating the completion of the calibration operation started by the depression of the calibration switch are displayed together on the display screen 4A, for example, as shown in FIG. 9. FIG. 9 illustrates one example of the image and the character string after the image rotation processing according to the second embodiment has been performed.

As described above, according to the endoscope system 102 of the present embodiment, when the insertion assisting instrument is used with the insertion portion 11 being inserted through the inner portion of the flexible tube portion 21, it is possible to cause the direction in which the angle of the distal end portion 212 is changed in response to the operation of the angle operation portion 22 to match with the direction in which the field of view of the scanning endoscope 1 moves as the angle of the distal end portion 212 is changed. That is, according to the present embodiment, it is possible to improve the operability at the time of changing the angle of the insertion assisting instrument used with the endoscope being inserted therethrough.

### (Third Embodiment)

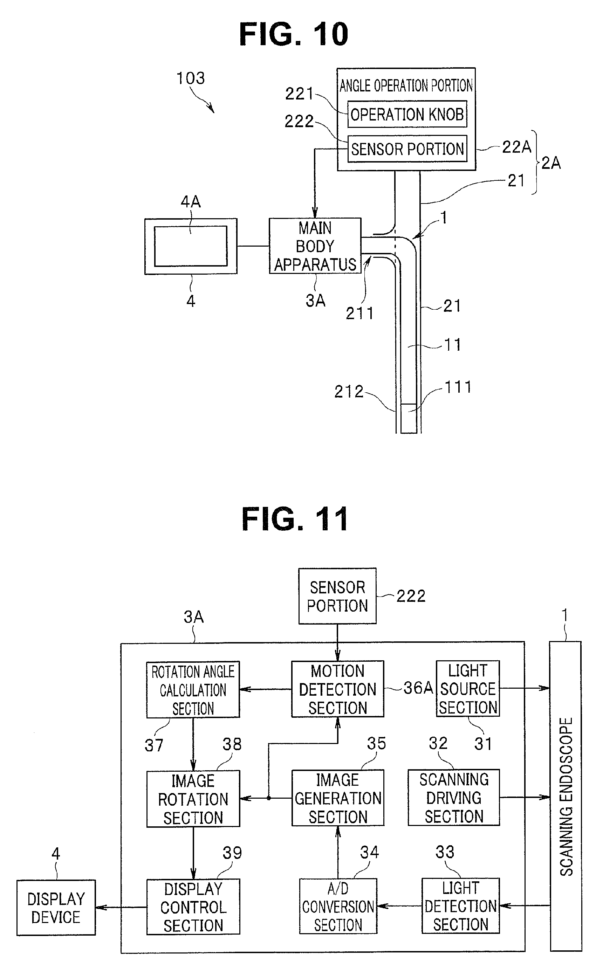

FIGS. 10 and 11 relate to the third embodiment of the present invention. FIG. 10 illustrates a configuration of a main part of an endoscope system according to the third embodiment. FIG 11 is a block diagram for illustrating one example of a configuration of a main body apparatus according to the third embodiment.

Note that, in the present embodiment, detailed description on the parts having configurations same as those in at least either the first or second embodiment will be appropriately omitted and description will be mainly made on the parts having configurations different from those in both of the first and second embodiments.

An endoscope system 103 includes a scanning endoscope 1, an insertion assisting instrument 2A, a main body apparatus 3A, and a display device 4, as shown in FIGS. 10 and 11.

The insertion assisting instrument 2A includes a flexible tube portion 21 and an angle operation portion 22A including an operation knob 221 and a sensor portion 222.

The operation knob 221 includes a first knob (not shown) with which an operation for changing the angle of the distal end portion 212 in the up and down directions can be performed, and a second knob (not shown) with which an operation for changing the angle of the distal end portion 212 in the right and left directions can be performed, for example.

The sensor portion 222 includes a rotary position sensor, etc., for example, and configured to be able to separately output voltages corresponding to the rotation angles of the first knob and the second knob of the operation knob 221.

When detecting that the angle of the distal end portion 212 has been changed in a predetermined direction on the basis of the voltage outputted from the sensor portion 222, the motion detection section 36A performs the same processing (pattern recognition, template matching, or the like) as that described in the second embodiment, to thereby obtain the motion vector of the images outputted from the image generation section 35. In addition, the motion detection section 36A outputs the motion vector obtained by the above-described processing to the rotation angle calculation section 37.

The rotation angle calculation section 37 performs the same processing as that described in the second embodiment, to thereby calculate an angle θ3 indicating to what extent the motion vector outputted from the motion detection section 36A is rotated with respect to the reference direction to be described later. In addition, the rotation angle calculation section 37 determines whether or not the angle θ3 obtained by the above-described processing is larger than the threshold θTH. When obtaining the determination result that the angle θ3 is larger than the threshold θTH, the rotation angle calculation section 37 outputs the angle θ3 to the image rotation section 38. On the other hand, when obtaining the determination result that the angle θ3 is equal to or smaller than the threshold θTH, the rotation angle calculation section 37 does not output the angle θ3 to the image rotation section 38, and calculates the angle θ3 of the motion vector to be outputted next from the image recognition section 36.

When the angle θ3 is outputted from the rotation angle calculation section 37, the image rotation section 38 performs image rotation processing on the image outputted from the image generation section 35 for displaying, on the display screen 4A of the display device 4, the image with the angle θ3 being offset. In other words, the image rotation section 38 performs, on the basis of the angle θ3 outputted from the rotation angle calculation section 37, the image rotation processing for rotating the image outputted from the image generation section 35 by negative θ3.

The display control section 39 performs processing on the image outputted from the image rotation section 38 for adapting the display format of the image to a predetermined display format, to output the image subjected to the processing to the display device 4.

Next, description will be made on the working of the endoscope system 103 according to the present embodiment. Note that, hereinafter, for simplification, description will be made by taking the case where the processing for obtaining the motion vector is performed when the motion detection section 36A detects that the angle of the distal end portion 212 has been changed in the up direction, as an example.

The user inserts the insertion portion 11 into the inner portion of the flexible tube portion 21 from the insertion port 211 of the insertion assisting instrument 2A in the state where the scanning of the object by the scanning endoscope 1 and the image generation by the main body apparatus 3A are started.

After that, the user operates the first knob of the operation knob 221 in the state where the distal end portion 111 is protruded from the distal end portion 212, to thereby change the angle of the distal end portion 212 in the up direction. Then, in accordance with such an operation, voltage corresponding to the rotation angle of the first knob is outputted from the sensor portion 222.

When detecting that the angle of the distal end portion 212 has been changed in the up direction on the basis of the voltage outputted from the sensor portion 222, the motion detection section 36A performs the same processing (processing such as pattern recognition or template matching) as that described in the second embodiment, to thereby obtain the motion vector of the images outputted from the image generation section 35 and output the obtained motion vector to the rotation angle calculation section 37.

The rotation angle calculation section 37 performs the same processing as that described in the second embodiment, to thereby calculate the angle θ3 indicating to what extent the motion vector outputted form the image recognition section 36 is rotated with respect to the down direction (reference direction) on the display screen 4A. In addition, the rotation angle calculation section 37 determines whether or not the angle θ3 obtained by the above-described processing is larger than the threshold θTH. When obtaining the determination result that the angle θ3 is larger than the threshold θTH, the rotation angle calculation section 37 outputs the angle θ3 to the image rotation section 38. On the other hand, when obtaining the determination result that the angle θ3 is equal to or smaller than the threshold θTH, the rotation angle calculation section 37 does not output the angle θ3 to the image rotation section 38 and calculates the angle θ3 of the motion vector to be outputted next from the image recognition section 36.

The image rotation section 38 performs, on the basis of the angle θ3 outputted from the rotation angle calculation section 37, the image rotation processing for rotating the image outputted from the image generation section 35 by negative θ3.

That is, according to the operations of the rotation angle calculation section 37 and the image rotation section 38 as described above, only when the angle θ3 calculated by the rotation angle calculation section 37 is larger than the threshold θTH, the image rotation processing is performed on the image outputted from the image generation section 35 by the image rotation section 38. In addition, according to the operations of the rotation angle calculation section 37 and the image rotation section 38, only in the case where the mismatch amount between the direction in which the angle of the distal end portion 212 is changed in response to the operation of the operation knob 221 and the direction in which the field of view of the scanning endoscope 1 moves in accordance with the change of the angle of the distal end portion 212 is larger than a predetermined mismatch amount (represented by the threshold TH, for example), the image rotation processing is performed on the image generation section 35.

Note that the endoscope system 103 according to the present embodiment may have another configuration different from the one in which the voltage outputted from the sensor portion 222 provided in the angle operation portion 22 is inputted to the motion detection section 36A, as long as the endoscope system is configured to be able to detect that the angle of the distal end portion 212 has been changed in a predetermined direction. Specifically, the endoscope system 103 according to the present embodiment may be configured such that output from a stress sensor provided in the bending portion of the flexible tube portion 21, such as a pressure sensitive conductive rubber, a capacitive pressure sensor or a piezoelectric sensor is inputted to the motion detection section 36A, for example. Alternatively, the endoscope system 103 according to the present embodiment may be configured such that a detection result obtained by a shape detection system for detecting the shape of the flexible tube portion 21 is inputted to the motion detection section 36A, for example.

As described above, the endoscope system 103 of the present embodiment enables the direction in which the angle of the distal end portion 212 is changed in response to the operation of the operation knob 221 to match with the direction in which the field of view of the scanning endoscope 1 moves as the angle of the distal end portion 212 is changed, when the insertion assisting instrument is used with the insertion portion 11 being inserted through the inner portion of the flexible tube portion 21. That is, according to the present embodiment, it is possible to improve the operability at the time of changing the angle of the insertion assisting instrument used with the endoscope being inserted therethrough.

### (Fourth Embodiment)

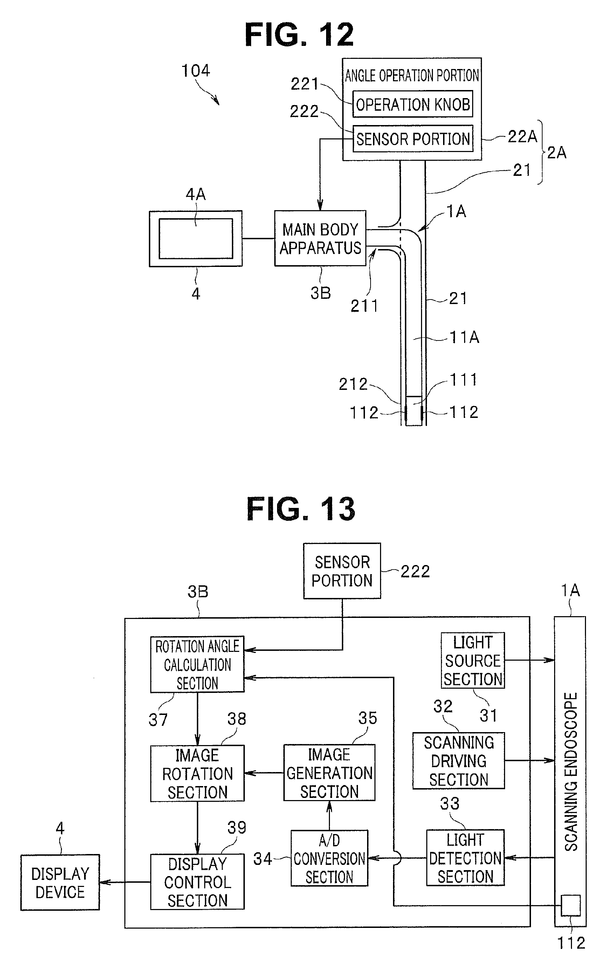

FIGS. 12 and 13 relate to the fourth embodiment of the present invention. FIG 12 illustrates a configuration of a main part of an endoscope system according to the fourth embodiment.

Note that, in the present embodiment, detailed description on the parts having configurations same as those in at least one of the first to third embodiments will be appropriately omitted and description will be mainly made on the parts having configurations different from those in all of the first to third embodiments.

An endoscope system 104 includes a scanning endoscope 1A, an insertion assisting instrument 2A, a main body apparatus 3B, and a display device 4, as shown in FIG 12.

The scanning endoscope 1A includes an insertion portion 11A which substantially corresponds to the one configured by adding a sensor portion 112 to the insertion portion 11 of the scanning endoscope 1.

The sensor portion 112 is provided with four stress sensors arranged so as to be able to detect the extension/contraction state in the longitudinal direction of the distal end portion 111 (of the insertion portion 11) in association with the respective up, down, right, and left directions at the time when the optical image obtained by scanning by the scanning endoscope 1A is displayed as an image on the display screen 4A. Specifically, the above-described stress sensors are configured by a pressure sensitive conductive rubber, or a capacitive stress sensor, for example. Furthermore, the sensor portion 112 is configured to be able to output the detection result of the extension/contraction state in the longitudinal direction of the distal end portion 111 as an electric parameter such as a resistance value to the main body apparatus 3B.

FIG. 13 is a block diagram for illustrating one example of the configuration of the main body apparatus according to the fourth embodiment.

As shown in FIG 13, the main body apparatus 3B includes a light source section 31, a scanning driving section 32, a light detection section 33, an A/D conversion section 34, an image generation section 35, a rotation angle calculation section 37, an image rotation section 38, and a display control section 39.

The image generation section 35 performs processing such as two-dimensional mapping on the digital signal outputted from the A/D conversion section 34 in a time-series manner, to thereby generate an image corresponding to the field of view of the scanning endoscope 1 and output the generated image to the image rotation section 38.

The rotation angle calculation section 37 detects the deformation state of the insertion portion 11A inserted through the inner portion of the flexible tube portion 21, estimates the orientation of the image to be outputted from the image generation section 35 on the basis of the detected deformation state, and calculates the angle indicating to what extent the estimated orientation of the image is rotated, with the predetermined direction as the reference direction, when the angle of the distal end portion 212 has been changed in a predetermined direction in response to the operation of the angle operation portion 22. Specifically, the rotation angle calculation section 37, for example, detects that the angle of the distal end portion 212 has been changed in the predetermined direction on the basis of the voltage outputted from the sensor portion 222, detects the extension/contraction state in the longitudinal direction of the distal end portion 111 on the basis of the electric parameter outputted from the sensor portion 112, and estimates the orientation of the image to be outputted from the image generation section 35 on the basis of the detected extension/contraction state. Then, the rotation angle calculation section calculates an angle θ4 indicating to what extent the estimated orientation of the image is rotated, with the predetermined direction as the reference direction, and outputs the calculated angle θ4 to the image rotation section 38.

When the angle θ4 is outputted from the rotation angle calculation section 37, the image rotation section 38 performs image rotation processing on the image outputted from the image generation section 35 for displaying, on the display screen 4A of the display device 4, the image with the angle θ4 being offset. In other words, the image rotation section 38 performs, on the basis of the angle θ4 outputted from the rotation angle calculation section 37, the image rotation processing for rotating the image outputted from the image generation section 35 by negative θ4.

The display control section 39 performs processing on the image outputted from the image rotation section 38 for adapting the display format of the image to the predetermined display format, and outputs the image subjected to the processing to the display device 4.

Next, the working of the endoscope system 104 according to the present embodiment will be described. Note that, hereinafter, for simplification, description will be made by taking the case where the angle of the distal end portion 212 has been changed in the up direction, as an example.

The user inserts the insertion portion 11A into the inner portion of the flexible tube portion 21 from the insertion port 211 of the insertion assisting instrument 2A in the state where the scanning of the object by the scanning endoscope 1A and image generation by the main body apparatus 3 are started.

After that, the user operates the first knob of the operation knob 221 in the state where the distal end portion 111 is protruded from the distal end portion 212, to thereby change the angle of the distal end portion 212 in the up direction. Then, in accordance with such an operation, voltage corresponding to the rotation angle of the first knob is outputted from the sensor portion 222. In addition, in accordance with the above-described operation, an electric parameter corresponding to the extension/contraction state in the longitudinal direction of the distal end portion 111 is outputted from the sensor portion 112.

The rotation angle calculation section 37 detects, on the basis of the voltage outputted from the sensor portion 222, that the angle of the distal end portion 212 has been changed in the up direction in response to the operation of the operation knob 221. In addition, the rotation angle calculation section 37 detects, on the basis of the electric parameter outputted from the sensor portion 112, the extension/contraction state in the longitudinal direction of the distal end portion 111, and further estimates the orientation of the image generated by the image generation section 35 on the basis of the detected extension/contraction state. Then, the rotation angle calculation section 37 calculates the angle θ4 indicating to what extent the orientation of the image estimated as described above is rotated with respect to the reference direction, when the up direction of the angle of the distal end portion 212 is defined as the reference direction, and outputs the calculated angle θ4 to the image rotation section 38.

The image rotation section 38 performs, on the basis of the angle θ4 outputted from the rotation angle calculation section 37, image rotation processing on the image outputted from the image generation section 35 for rotating the image by negative θ4.

That is, according to the above-described operations of the rotation angle calculation section 37 and the image rotation section 38, when the angle of the distal end portion 212 is changed in a predetermined direction of the up, down, right, and left directions in response to the operation of the operation knob 221, image rotation processing is performed for changing the up, down, right, or left direction of the image outputted from the image generation section 35 in accordance with the predetermined direction.

Note that the endoscope system 104 according to the present embodiment may have another configuration different from the one in which the electric parameter outputted from the sensor portion 112 provided in the distal end portion 111 is inputted to the rotation angle calculation section 37, as long as the endoscope system is configured to be able to detect the deformation state of the insertion portion 11 A inserted through the inner portion of the flexible tube portion 21. Specifically, the endoscope system 104 according to the present embodiment may be configured such that output from four photodetectors is inputted to the rotation angle calculation section 37, for example, the four photodetectors being arranged so as to be able to detect the light leaking out from the optical fiber of the light-guiding portion of the insertion portion 11A in association with the up, down, right, and left directions of the image obtained by scanning by the scanning endoscope 1A. Alternatively, the endoscope system 104 according to the present embodiment may be configured such that the detection result obtained by the shape detection apparatus for detecting the shape of the insertion portion 11A is inputted to the rotation angle calculation section 37, for example.

As described above, the endoscope system 104 according to the present embodiment enables the direction in which the angle of the distal end portion 212 is changed in response to the operation of the operation knob 221 to match with the direction in which the field of view of the scanning endoscope 1A moves as the angle of the distal end portion 212 is changed, when the insertion assisting instrument is used with the insertion portion 11 A being inserted through the inner portion of the flexible tube portion 21. That is, according to the present embodiment, it is possible to improve the operability at the time of changing the angle of the insertion assisting instrument used with the endoscope being inserted therethrough.

Note that each of the embodiments can be applied not only to a system including a scanning endoscope but also to a system including another endoscope such as a fiber scope by appropriately modifying the configurations of the endoscope systems 101 to 104, for example.

The present invention is not limited to each of the above-described embodiments, and it is needless to say that various changes and modifications are possible without departing from the gist of the invention.

This application claims the benefit of Japanese Application No. 2013-146852 filed in Japan on July 12, 2013, and the above described disclosure is incorporated by reference in the present description, claims, and drawings.

## Claims

1. An endoscope system comprising:
an endoscope including an insertion portion formed so as to have flexibility and an elongated shape, and configured to have a field of view in front of a distal end portion of the insertion portion;
an insertion assisting instrument including a conduit having an inner portion through which the insertion portion can be inserted, and the insertion assisting instrument being configured to enable an angle of a distal end portion of the conduit to be changed in response to an operation of an angle operation portion;
an image generation section configured to generate an image corresponding to the field of view of the endoscope and output the generated image;
a rotation angle calculation section configured to calculate a rotation angle indicating to what extent an orientation of the image is rotated with respect to a reference direction, the image being outputted from the image generation section when at least a part of the insertion portion is inserted through the conduit; and
an image rotation section configured to perform image rotation processing on the image outputted from the image generation section, for displaying, on a display screen of a display section, the image with the rotation angle being offset.

2. The endoscope system according to claim 1, wherein
the conduit includes a predetermined mark drawn on the inner portion thereof, the predetermined mark enabling recognition that the angle of the distal end portion of the conduit is changed in a predetermined direction in response to the operation of the angle operation portion, and
the rotation angle calculation section calculates, as the rotation angle, an angle indicating to what extent a position of the predetermined mark included in the image outputted from the image generation section is rotated, with a direction matching with the predetermined direction on the display screen as the reference direction.

3. The endoscope system according to claim 1, further comprising
a motion detection section configured to obtain a motion vector of an object included in the image outputted from the image generation section when the angle of the distal end portion of the conduit is changed in a predetermined direction in response to the operation of the angle operation portion, wherein
the rotation angle calculation section calculates, as the rotation angle, an angle indicating to what extent the motion vector obtained by the motion detection section is rotated, with a direction opposite to the predetermined direction on the display screen as the reference direction.

4. The endoscope system according to claim 3, wherein only when the angle calculated by the rotation angle calculation section is larger than a predetermined threshold value, the image rotation processing is performed on the image outputted from the image generation section by the image rotation section.

5. The endoscope system according to claim 1,
wherein, when the angle of the distal end portion of the conduit is changed in a predetermined direction in response to the operation of the angle operation portion, the rotation angle calculation section detects a deformation state of the insertion portion inserted through the inner portion of the conduit, estimates an orientation of the image generated by the image generation section based on the detected deformation state, and calculates, as the rotation angle, an angle indicating to what extent the estimated orientation of the image is rotated, with the predetermined direction as the reference direction.

6. The endoscope system according to claim 1, wherein the endoscope is configured to obtain an optical image by scanning an object which is present in front of the distal end portion.
